Europäisches Patentamt

European Patent Office

Office européen des brevets

(19)

(11) Numéro de publication: **0 255 794**
**B1**

(12) **FASCICULE DE BREVET EUROPEEN**

(45) Date de publication du fascicule du brevet:
**07.11.90**

(21) Numéro de dépôt: **87420194.0**

(22) Date de dépôt: **09.07.87**

(51) Int. Cl.⁵: **C07C 51/10,** C07C 65/24,
C07C 51/145

(54) **Procédé de préparation d'acide aromatique.**

(30) Priorité: **25.07.86 FR 8610991**
**25.07.86 FR 8610992**
**25.07.86 FR 8610993**

(43) Date de publication de la demande:
**10.02.88 Bulletin 88/6**

(45) Mention de la délivrance du brevet:
**07.11.90 Bulletin 90/45**

(84) Etats contractants désignés:
**AT BE CH DE ES FR GB GR IT LI LU NL SE**

(56) Documents cités:
**EP-A- 0 034 292**
**FR-A- 2 195 612**
**FR-A- 2 274 595**

(73) Titulaire: **RHONE-POULENC CHIMIE, 25, quai Paul
Doumer, F-92408 Courbevoie Cédex(FR)**

(72) Inventeur: **Biuthe, Norbert, 39, rue Clément Michut,
F-69100 Villeurbanne(FR)**
Inventeur: **Perron, Robert, La Pecolière,
F-69390 Charly(FR)**

(74) Mandataire: **Le Pennec, Magali et al, RHONE-POULENC
INTERSERVICES Service Brevets Chimie 25, Quai Paul
Doumer, F-92408 Courbevoie Cédex(FR)**

ACTORUM AG

**Description**

La présente invention concerne un nouveau procédé de préparation d'acides aromatiques. Elle concerne plus particulièrement un procédé amélioré de production d'acides aromatiques par hydroxycarbonylation d'halogénures aromatiques en présence d'un métal noble, d'une base azotée tertiaire et d'un agent complexant à base de phosphore.

Parmi les divers procédés connus de préparation d'acide aromatique on peut considérer deux brevets récents.

Il est ainsi connu d'après le brevet US 4 034 004 un procédé de préparation d'acides carboxyliques par réaction en milieu alcalin d'halogénures organiques aromatiques ou aliphatiques avec du monoxyde de carbone en utilisant comme catalyseurs des complexes phosphiniques du palladium et des sels d'alkyl-ammonium quaternaires. La réaction a lieu en milieu biphasique ce qui implique la présence de l'agent de transfert de phase qu'est le sel d'alkylammonium quaternaire. La phase aqueuse est composée d'une base alcaline minérale et du sel d'ammonium quaternaire. Il est précisé dans le texte que la réaction doit avoir lieu à une température comprise entre 50 et 150°C. Au-dessus de cette température et même au-delà de 120°C le monoxyde de carbone réagit avec la base alcaline pour donner naissance à du formiate de sodium qui consomme inutilement, la base alcaline et l'oxyde de carbone introduit, abaissant fortement la productivité.

Dans ce brevet (exemple 2) est décrit un exemple de recyclage du catalyseur palladium/phosphine. Au cours du premier recyclage le rendement en acide aromatique obtenu baisse d'environ 10 %. Ainsi le procédé n'est pas extrapolable à plus de 4 à 5 recyclages successifs.

Il est également connu d'après le brevet EP 34 292 un procédé de préparation d'acide anthranilique par mise en contact de monoxyde de carbone, d'eau, d'une trialyklamine, d'un catalyseur à base de palladium complexé par une phosphine et d'un acylamino, iodo ou bromobenzène. La quantité d'eau mise en oeuvre dans ce procédé est très faible et ne permet pas d'avoir un système biphasique mais uniquement une phase homogène très visqueuse.

Cette méthode ne permet pas une séparation facile du produit de réaction et du catalyseur et ne permet pas ainsi un recyclage aisé de ce dernier. Cette méthode applicable à des produits ou des intermédiaires pharmaceutiques à forte valeur ajoutée n'est pas transposable à des produits de plus grande consommation.

La présente invention a permis d'améliorer les procédés déjà connus de façon à pouvoir disposer d'un procédé industriellement exploitable et économiquement rentable pour la préparation d'acides aromatiques permettant le recyclage du catalyseur. Procédé mettant en contact un halogénure aromatique dont le ou les halogènes sont l'iode et/ou le brome en solution dans un solvant organique avec de l'oxyde de carbone et de l'eau en présence d'un catalyseur à base de palladium, d'une base organique azotée tertiaire et d'un catalyseur à base de palladium, d'une base organique azotée tertiaire et d'un agent complexant le palladium choisi parmi les phosphines ou les phosphites, caractérisé en ce que le milieu réactionnel est constitué d'une phase organique et d'une phase aqueuse, la phase aqueuse étant en quantité suffisante pour pouvoir dissoudre les sels de la base organique azotée tertiaire avec les acides dégagées par la réaction.

La réaction chimique mise en jeu dans le présent procédé peut se résumer ainsi :

$$Ar\ X + CO + H_2O + 2NR_3 \rightarrow Ar\ COONHR_3 + X\ NHR_3$$

La quantité d'eau minimale pour opérer en milieu biphasique doit assurer la solubilité des sels de l'acide aromatique et de l'hydracide libéré avec la base organique. La quantité minimale d'eau sera fonction de l'acide aromatique formé. Ainsi pour l'acide paraphénoxybenzoïque il est préférable d'utiliser un maximum de 3, 5 moles de bromodiphényléther par litre d'eau.

Les halogénures aromatiques répondent de préférence à la formule générale suivante (I) :

$$(X)_n - Ar - (R_1)_{nl}\ (I)$$

dans laquelle :
- X représente l'iode ou le brome
- Ar représente un radical mono ou polycyclique ou hétérocyclique
- n est égal à 1 ou 2 par cycle
- nl est un nombre entier supérieur ou égal à 1 et inférieur ou égal à 4
- $R_1$ représente un ou plusieurs groupes identiques ou différents choisi parmi les radicaux : hydrogéno, fluoro, chloro, nitrile, alkyle, alkenylène, alcoxy, cycloalkyle, cycloalcoxy, alkyl carbonyloxy, cycloalkylcarbonyloxy, aryl, aryloxy, arylcarbonyloxy, aryloxycarbonyl, les motifs alkyl et aryl ayant de 1 à 20 atomes de carbone et étant éventuellement substitués par un ou plusieurs atomes de fluor et/ou de chlore.

Dans la formule générale (I) Ar représente un radical mono ou polycyclique. Parmi les radicaux polycycliques on peut notamment utiliser les cycles condensés tels que le naphtalène, l'anthracène ou des cycles reliés entre eux par une liaison covalente tel que le biphényle ou reliés par un hétéroatome, par exemple l'oxygène ou le soufre tel que l'oxyde de biphényle.

Parmi les composés de formule (I) on préfère mettre en oeuvre ceux pour lesquels Ar représente un motif benzénique ou un motif diphényléther et ceux pour lesquels $R_1$ représente :

- un groupe alkyl ou alcoxy ayant 1 à 4 atomes de carbone tel que les radicaux méthyl, éthyl, propyl, butyl, trifluorométhyl, difluorochlorométhyl, méthoxy, éthoxy, propyloxy, butoxy, trifluorométhoxy, trichlorométhoxy,
- un groupe cycloalkyl ou cycloalcoxy ayant 5 à 10 atomes de carbone tel que les radicaux cyclopentyl, cyclohexyl, cyclooctyl,
- un groupe aryl ou aryloxy tel que les radicaux phényl, xylyl, toluyl, méthoxyphényl, éthoxyphényl, phénoxy, méthylphénoyloxy, diméthylphényloxy,
- un groupe alcoxycarbonyl ou alkylcarbonyloxy dont le groupe alkyl a de préférence 1 à 4 atomes de carbone,
- un groupe cycloalcoxycarbonyl ou cycloalkylcarbonyloxy tel que les radicaux cyclopentyloxycarbonyl, cyclohexyloxycarbonyl.

Comme exemples de composés de formule (I) on peut citer : le bromobenzène, les bromotoluènes, les diméthylbromobenzènes, les fluorobromobenzènes, les fluoroiodobenzènes, les difluorobromobenzènes, les trifluorométhylbromobenzènes, les trifluorométhyliodobenzènes, les difluorochlorométhylbromobenzènes, les difluorochlorométhoxybromobenzènes, les bromobenzonitriles, les dibromobenzènes, les diiodobenzènes, les bromonaphtalènes, les bromopyridines, les bromodiphényléthers, les bromobenzoates de méthyle, les bromoanisoles, les bromophénétoles, les diméthoxybromobenzènes, les triméthoxy-3,4,5 bromobenzènes, le bromo-3 méthylènendioxybenzène, les dibromodiphényléthers.

On préfère mettre en oeuvre le p-bromodiphényléther et le dibromo-4,4′ diphényléther.

Le catalyseur à base de palladium utilisé est choisi parmi les sels d'acides minéraux ou organiques ou leurs complexes avec des composés donneurs de doublets électroniques, en particulier avec les phosphines, les phosphites ou les arsines.

Comme exemples spécifiques de dérivés du palladium, on peut citer les carboxylates (acétate, propionate, butyrate, benzoate) de palladium (II) et les complexes du palladium de formules générales $PdX_2|P(R)_3|_2$ ou $PdX_2|P(OR)_3|_2$ dans lesquelles X représente un atome d'halogène (brome, chlore) ou une reste d'un acide minéral ou carboxylique et R un radical hydrocarboné. On peut citer en particulier le dichlorodi(triphénylphosphino)palladium (II) et le dichlorodi(tritolylphosphino)palladium (II).

On peut citer comme exemples non limitatifs de phosphines ou phosphites : la triphénylphosphine, le triphénylphosphite, le diéthylphénylphosphine, le diéthylphénylphosphite, la tritolylphosphine, le tritolylphosphite, la trinaphtylphosphine, le trinaphtylphosphite, la diphénylméthylphosphine, le diphénylméthylphosphite, la diphénylbutylphosphine, le diphénylbutylphosphite, la tris-(p-méthoxycarbonylphényl)-phosphine, le tris-(p-méthoxycarbonylphényl)-phosphite, la tris-(p-cyanophényl)-phosphine, le tris-(cyanophényl)-phosphite, le triéthylphosphite, la tributylphosphine, le tributylphosphite.

La présence d'un agent complexant phosphoré libre dans le milieu réactionnel dépend de la nature du cataylseur et/ou des conditions de la réaction. Lorsque le catalyseur est constitué par un complexe d'un métal noble avec une phosphine ou un phosphite, la présence de ces derniers à l'état libre n'est pas indispensable. Toutefois elle s'avère avantageuse lorsque la réaction est conduite à température élevée, par exemple à une témpérautre supérieure à 150°C. Lorsqu'on met en oeuvre un métal noble à l'état métallique ou un dérivé non complexé par une phosphine ou un phosphite tel que les carboxylates de métaux nobles par exemple, il est nécessaire d'opérer en présence du composé phosphoré.

La quantité de catalyseur exprimée en atome-gramme de métal ou en mole de dérivé métallique par mole d'halogénure aromatique peut varier dans de larges limites. Ainsi elle peut être comprise entre $10^{-5}$ et $10^{-1}$ at.g ou mole par mole et de préférence entre $10^{-4}$ et $10^{-2}$ at.g ou mole par mole.

Lorsqu'on conduit la réaction en présence d'un phosphite ou d'une phosphine la quantité en est choisie de façon à ce que le rapport du nombre d'atome-gramme de phosphore (P) au nombre d'atome-gramme de métal (M) soit au moins égal à 2. Le rapport P/M peut prendre des valeurs aussi élevées que 10 000. Un rapport P/M compris entre 5 et 1000 est en général convenable.

A titre de base azotée tertiaire, on peut faire appel à des amines de formule générale :

$N - (R_1)_3$

dans laquelle les radicaux $R_1$, identiques ou différents, représentent des restes hydrocarbonés comportant de 1 à 20 atomes de carbone tels que les radicaux alkyle, cycloalkyle ou aryle. De préférence les symboles $R_1$ représentent des radicaux alkyles comportant de 1 à 10 atomes de carbone ou des radicaux cycloalkyles ayant de 5 à 10 atomes de carbone. Comme exemple de telles bases on peut citer la triéthylamine, la tri-n-propylamine, la tri-n-butylamine, la méthyldibutylamine, la méthyldicyclohexylamine, l'éthyldiisopropylamine. On peut encore faire appel à des bases tertiaires hétérocycliques telles que la pyridine et les picolines. On préfère tout particulièrement utiliser la triéthylamine.

La quantité de base doit être suffisante pour neutraliser l'acide aromatique formé et l'hydracide libéré par la réaction, c'est-à-dire qu'elle doit être d'au moins 2 moles par équivalent d'halogène réactif dans la molécule d'halogénure aromatique. On entend par halogène réactif le brome et/ou l'iode.

Il n'y pas de limite supérieure à la quantité de base introduite. Elle sera simplement adaptée par l'homme de l'art à l'économie du procédé.

Le procédé de la présente invention est conduit en milieu biphasique. Le milieu organique est constitué de l'halogénure aromatique, du palladium, de la phosphine, de la base organique et d'un solvant. Le solvant est choisi parmi les solvants inertes dans les conditions de la réaction et non miscibles à l'eau. On peut utiliser des hydrocarbures aliphatiques ou cycloaliphatiques saturés (hexane, cyclohexane) ou aromatiques : benzène, toluène, xylène, halogénobenzène tel que le chlorobenzène, des esters tels que le benzoate de méthyle, le téréphtalate de méthyle, l'adipate de méthyle, le phtalate de dibutyle, des éthers aromatiques et parmi eux le diphényléther qui est utilisé préférentiellement lorsque l'on veut préparer l'acide phénoxybenzoïque.

Parmi ces solvants on préfère utiliser le chlorobenzène et le diphényléther.

L'utilisation de ce type de solvant présente au moins deux avantages par rapport aux solvants couramment utilisés dans les réactions de carbonylation dans l'art antérieur.

En effet, d'une part, la productivité, c'est-à-dire la quantité d'acide aromatique produite par heure et par litre de solution est augmentée par rapport à la même réaction réalisée dans un solvant alkylbenzénique tel que le toluène.

D'autre part, ces solvants permettent de diminuer la quantité d'agent complexant du palladium, notamment de la phosphine, car ils assurent une meilleure stabilité du catalyseur et permettent ainsi un recyclage beaucoup plus aisé de ce dernier.

Les acides aromatiques obtenus par le procédé de la présente invention répondent à la formule générale (II) :

$(R_1)_{nl}$ - Ar $(COOH)_n$

dans laquelle Ar, n, nl et $R_1$ ont la même signification que prédédemment.

On peut citer parmi les composé de formule (II) : lers acides benzoïques, chlorobenzoïques, toluïques, méthoxybenzoïques, trifluorométhoxybenzoïques, trifluorométhylbenzoïques, phénoxybenzoïques, les diacides tels que les acides phtaliques, le 4,4' dihydroxycarbonyldiphényléther.

La température à laquelle est mis en oeuvre le procédé selon l'invention peut varier dans de larges limites. On préfère cependant maintenir une température comprise entre 50°C et 250°C et tout particulièrement une température comprise entre 100 et 200°C.

La pression à laquelle peut être mis en oeuvre le présent procédé peut varier dans de larges limites. Néanmoins contrairement à ce qu'attendait l'homme de l'art la diminution de la pression du monoxyde de carbone au cours de la réaction améliore la productivité au lieu de la diminuer. Le monoxyde de carbone étant un des réactants l'augmentation de pression aurait dû accélérer la réaction au lieu de la ralentir. Il a été trouvé de façon tout-à-fait surprenante que la conclusion établie en chimie qu'une réaction entre un gaz et un liquide était accélérée par la pression était fausse dans le cas de la présente réaction.

Pour obtenir une vitesse de réaction élevée il faut diminuer la quantité de monoxyde de carbone dissoute dans le milieu réactionnel. Ainsi il est préférable de maintenir la pression partielle de monoxyde de carbone au maximum à 5 bars. La pression réactionnelle totale variable avec la température et les produits mis en oeuvre sera adaptée par l'homme de l'art de façon à maintenir une pression partielle de monoxyde de carbone encore plus préférentiellement inférieure ou égale à 3 bars.

D'un point de vue pratique le procédé selon l'invention est mis en oeuvre par introduction dans un autoclave inerte de l'halogénure d'aryle, de la base azotée, du catalyseur et le cas échéant du dérivé phosphoré et d'un solvant puis alimentation dans l'autoclave fermé d'une pression adéquate de monoxyde de carbone. Le contenu de l'autoclave est ensuite porté sous agitation à la température convenable jusqu'à ce que l'absorption de gaz cesse. En fin de réaction le contenu de l'autoclave est refroidi, l'autoclave dégazé et la phase aqueuse contenant l'acide aromatique neutralisé par la base organique est séparée. La phase organique contenant le catalyseur et l'agent complexant peut être recyclée pour une nouvelle opération. L'acide libre est obtenu par précipitation à partir de la phase aqueuse après traitement avec un acide minéral.

Le procédé selon l'invention peut être mis en oeuvre en continu ou en discontinu.

Les exemples suivants illustrent l'invention et montrent comment elle peut être mise en pratique.

## EXEMPLES

### EXEMPLE 1

Dans un réacteur en acier inoxydable (marque commerciale Hastelloy B2) résistant à la pression, muni d'un dispositif de chauffage et agité par un agitateur de type CAVITATOR, on charge :
- 37,35 g de parabromophénoxybenzène (150 mmoles)
- 67,2 mg de palladium diacétate (0,3 mmole)
- 2,36 g de triphénylphosphine (9 mmoles)
- 33,33 g de triéthylamine (330 mmoles)
- 45 ml d'eau
- 30 ml de toluène

Le réacteur est purgé au monoxye de carbone puis on élève progressivement la température jusqu'à 130°C. A 100°C on introduit du monoxyde de carbone pour que la pression totale à 130°C dans le réacteur soit égale à 6 bar.

Le réacteur est relié à une réserve de monoxyde de carbone qui maintient la pression constante. On suit le degré d'avancement de la réaction par la chute de pression dans la réserve.

Après 1 h 45 mn de réaction l'absorption cesse. La vitesse initiale d'absorption est 150 mmoles.$h^{-1}$.

En fin de réaction on refroidit le réacteur, on laisse décanter puis on soutire la phase aqueuse.

On recharge ensuite les mêmes quantités de parabromophénoxybenzène, de triéthylamine et d'eau qui figurent ci-dessus.

On répète ainsi l'opération quatre fois. Les résultats sont rassemblés dans le tableau suivant :

| Recyclage | $\triangle$P (bar) | Durée de réaction | $V_i$ (mmole.h$^{-1}$) |
|-----------|--------------------|--------------------|------------------------|
| 1 | 22 | 1 h 45 mn | 155 |
| 2 | 23 | 1 h 30 mn | 197 |
| 3 | 24 | 1 h 15 mn | 218 |
| 4 | 22 | 1 h 30 mn | 197 |

Ces essais montrent qu'il est possible de recycler le catalyseur tout en conservant son activité. Ceci est rendu possible par le système biphasique utilisé qui permet de soutirer le produit de la réaction contenu dans la phase aqueuse sous pression de monoxyde de carbone tout en maintenant le système catalytique dans le réacteur.

EXEMPLE 2

On procédé comme dans l'exemple 1 mais on limite volontairement la première réaction à un taux de transformation (TT) incomplet.

| Essai | $\triangle$ P (bar) | Durée de réaction | $V_i$ (mmole.h$^{-1}$) |
|---|---|---|---|
| Essai initial TT env. 80 % | 17 | 50 mn | 170 |
| 1er recyclage | 22 | 1 h | 194 |
| 2e recyclage | 23 | 55 mn | 197 |
| 3e recyclage | 23 | 1 h | 203 |
| 4e recyclage | 23,5 | 55 mn | 200 |
| 5e recyclage | 23 | 50 mn | 256 |
| 6e recyclage | 24 | 45 mn | 280 |

Cette série d'essais montre qu'on peut recycler au moins 6 fois le catalyseur en maintenant au moins son activité initiale. Après le 4e recyclage on gagne même 25 % d'activité.

EXEMPLES 3

Dans un autoclave de 125 ml en Hastelloy [R] B2 on charge 12,45 g de parabromophénoxybenzène (50 mmoles), 22,4 mg de palladium diacétate (0,1 mmole), 524 mg de triphénylphosphine (2 mmoles) et 11,11 g de triéthylamine (110 mmoles).

On ajoute ensuite 10 ml de toluène et 15 ml d'eau.

Le réacteur est fermé puis purgé au monoxyde de carbone et agité par un système de va et vient. La température est progressivement amenée à 130°C et le monoxyde de carbone introduit dans le réacteur à 100°C pour que la pression totale à 130°C soit égale à 6 bar. Le réacteur est relié à une réserve de monoxyde de carbone qui maintient la pression constante dans le dit réacteur et à partir de laquelle on détermine l'absorption de monoxyde de carbone par la mesure de la chute de pression.

Après 2 h 20 mn de réaction l'absorption de monoxyde de carbone a cessé. Le réacteur est refroidi et dégazé. On sépare ensuite la phase organique et la phase aqueuse. Cette dernière est acidifiée par HCl 2 N jusqu'à pH = 2. L'acide précipité est filtré, lavé et séché. On obtient 10,34 g d'acide paraphénoxybenzoïque (rendement 97 %). La productivité en acide est de 89 g/heure/litre.

En fin de réaction on retrouve dans la phase organique 84 % du palladium théoriquement chargé.

EXEMPLE 4 à 6

On procède comme dans l'exemple 3 avec quelques modifications précisées dans le tableau suivant. Les résultats obtenus pour chaque essai figurent dans le même tableau.

| Exemple | Solvant | P$\phi_3$ (mmole) | Durée de réaction | Productivité (g/h/l) | Palladium (%) |
|---------|---------|------|------------|-------------|----------|
| 4 | Ph Cl | 2 | 1 h 50 mn | 115 | 100 |
| 5 | $\phi$o$\phi$ | 2 | 2 h | 100 | 73 |
| 6 | toluène | 4 | 5 h | 40 | 100 |

Le chlorobenzène amène un gain en productivité et permet par rapport aux autres solvants de minimiser la quantité de phosphine pour tenir le catalyseur en solution et ainsi le recycler.

EXEMPLE 7

Dans un autoclave de 125 ml en Hastelloy B2 on charge 224 mg de palladium diacétate (1 mmole), 1,31 g de triphénylphosphine (5 mmoles), 11,11 g de triéthylamine (110 mmoles) et 12,45 g (0,05 mole) de parabromophénoxybenzène. On ajoute ensuite 20 ml de toluène et 20 ml d'eau. L'autoclave après avoir été fermé est purgé avec du monoxyde de carbone. On élève la température jusqu'à 130°C l'autoclave étant agité par un système de va et vient. A 100°C on introduit le monoxyde de carbone pour que la pression totale à 130°C soit de 7 bar (la pression partielle de monoxyde de carbone est d'environ 4 bar).

Durant toute la durée de la réaction, le réacteur est relié à une réserve de monoxyde de carbone qui y maintient le pression constante. L'avancement de la réaction est suivi par la chute de presion de la réserve. Après 30 mn de réaction le produit de départ a été entièrement consommé. Le rendement en acide paraphénoxybenzoïque est quantitatif.

La vitesse initiale d'absorption mesurée à partir de la chute de pression de la réserve est de 56 bar.h$^{-1}$.

La productivité est de 340 g/heure/litre.

EXEMPLE 8 (exemple comparatif)

On procède comme dans l'exemple 7 mais la pression à 130°C dans le réacteur est maintenue à 30 bar (la pression partielle de monoxyde de carbone est de 27 bar). L'absorption de monoxyde de carbone cesse après 3 h de réaction et la vitesse initiale d'absorption est de 14 bar.h$^{-1}$.

Cet exemple montre qu'il est préférable de travailler à faible pression partielle de monoxyde de carbone.

La productivité est de 60 g/heure/litre.

EXEMPLE 9

On procède comme dans l'exemple 7 mais en chargeant 22,4 mg de palladium diacétate (0,1 mmole) et 1,05 g de triphénylphosphine. L'absorption de monoxyde de carbone s'arrête au bout de 5 h et la vitesse maximale d'absorption est de 6,4 bar.h$^{-1}$.

Le rendement en acide paraphénoxybenzoïque est quantitatif.

La productivité est de 34 g/heure/litre.

EXEMPLE 10

On procède comme dans l'exemple 9 mais à une température de 150°C. La pression partielle de monoxyde de carbone est de 3 bar. Après 50 mn de réaction le produit de départ est totalement transformé. La vitesse initiale d'absorption est 16,3 bar.h⁻¹.

La productivité est de 200 g/heure/litre.

EXEMPLE 11 (exemple comparatif avec l'exemple 9)

Dans un autoclave de 125 ml en hastelloy B2 on charge 22,4 mg de palladium diacétate (0,1 mmole), 524 mg de triphénylphosphine 11,11 g de triéthylamine (110 mmoles) et 12,45 g (0,05 mole) de parabromophénoxybenzène. La mise en oeuvre de la réaction est analogue à celle de l'exemple 1 sauf que la pression est maintenue à 4 bar pendant toute la durée de l'essai. La pression partielle de monoxyde de carbone est de 1 bar. Au bout de 4 h il n'y a plus d'absorption de monoxyde de carbone. La vitesse initiale d'absorption est de 6 bar.h⁻¹. En fin de réaction on décante et sépare les deux phases aqueuse et organique et la phase aqueuse est acidifiée avec HCl 2N jusqu'à pH = 2. L'acide paraphénoxybenzoïque ainsi précipité est filtré, lavé et séché. On obtient 10,59 g (rendement 99 %) d'un produit dont la pureté est au moins égale à 98 %.

La productivité est de 40 g/heure/litre.

Cet essai comparé à celui de l'exemple 9 montre que la pression partielle de monoxyde de carbone peut être inférieure à 1 bar sans que la productivité en soit affectée.

EXEMPLE 12

On procède comme dans l'exemple 10 mais en chargeant 11,2 mg de palladium diacétate (0,05 mmole), 10 ml de toluène et 15 ml d'eau.

Après 2 h 10 mn de réaction l'absorption de monoxyde de carbone cesse et le rendement en acide paraphénoxybenzoïque est quantitatif.

La productivité est de 100 g/heure/litre.

EXEMPLE 13

Dans un réacteur de 125 ml en hastelloy B2 on charge 112 mg de palladium diacétate (0,5 mmole), 524 mg de triphénylphosphine (2 mmoles), 18,5 g tributylamine (100 mmoles) et 6,2 g de dibromo-4,4' diphényléther (25 mmoles). On ajoute ensuite 10 ml de chlorobenzène et 15 ml d'eau. La mise en oeuvre est du même type que celles décrites précédemment. Après 2 h 30 mn de réaction à une pression totale de 10 bar et à 130°C l'absorption de monoxyde de carbone cesse. Après refroidissement du réacteur et dégazage on sépare la phase aqueuse de la phase organique. La phase aqueuse est acidifiée avec HCl 2N jusqu'à pH = 2. Le diacide précipité est filtré, lavé et séché. On obtient 4,53 g de produit (rendement 73 %).

La productivité est de 36 g/heure/litre.

**Revendications**

1. Procédé de préparation d'acide aromatique par mise en contact d'un halogénure aromatique dont le ou les halogènes sont l'iode et/ou le brome en solution dans un solvant organique avec de l'oxyde de carbone et de l'eau en présence d'un catalyseur à base de palladium, d'une base organique azotée tertiaire et d'un agent complexant le palladium choisi parmi les phosphines ou les phosphites caractérisé en ce qu'on opère en milieu liquide biphasique en l'absence d'agent de transfert de phase.

2. Procédé selon la revendication 1 caractérisé en ce que l'halogénure aromatique répond à la formule (I) :

$(X)_n - Ar - (R_1)_{nl}$ (I)

dans laquelle :

- X représente le chlore ou le brome
- Ar représente un radical mono ou polycyclique ou hétérocyclique
- n est égal à 1 ou 2 par cycle
- nl est un nombre entier supérieur ou égal à 1 et inférieur ou égal à 4
- $R_1$ représente un ou plusieurs groupes identiques ou différentes choisi parmi les radicaux : hydrogéno, fluoro, chloro, nitrile, alkyl, alkenylène, alcoxy, cycloalkyl, cycloalcoxy, alkylcarbonyloxy, cycloalkylcarbonyloxy, aryl, aryloxy, arylcarbonyloxy, aryloxycarbonyl, les motifs alkyl et aryl ayant de 1 à 20 atomes de carbone et étant éventuellement substitués par un ou plusieurs atomes de fluor et/ou de chlore.

3. Procédé selon la revendication 2 caractérisé en ce que dans la formule (I) Ar représente un motif benzénique ou diphényléther.

4. Procédé selon l'une quelconque des revendications 1 à 3 caractérisé en ce que parmi les composés de formule (I) on utilise le parabromodiphényléther et le dibromo 4'4-diphényléther.

5. Procédé selon la revendication 1 caractérisé en ce que le catalyseur est constitué par du palladium métallique ou un dérivé du palladium.

6. Procédé selon l'une quelconque des revendications 1 à 5, caractérisé en ce que la quantité de palladium exprimée en atome-gramme de métal noble ou en mole de dérivé métallique par mole d'halogénure aromatique est comprise dans un intervalle de $10^{-5}$ à $10^{-1}$.

7. Procédé selon l'une quelconque des revendications 1 à 6 caractérisé en ce que la quantité de dérivé phosphoré est telle que le rapport du nombre des atomes-grammes de phosphore au nombre des atomes-grammes de métal soit compris dans un intervalle de 1 à 10 000.

8. Procédé selon la revendication 1 caractérisé en ce que la base azotée tertiaire est la triéthylamine.

9. Procédé selon la revendication 1 caractérisé en ce que la température réactionnelle est comprise entre 100 et 250°C.

10. Procédé selon la revendication 1, caractérisé en ce que la pression partielle de monoxyde de carbone est inférieure à 5 bars.

11. Procédé selon la revendication 10, caractérisé en ce que la pression partielle de monoxyde de carbone est d'environ 3 bars.

12. Procédé selon la revendication 1 caractérise en ce que le solvant est choisi parmi les hydrocarbures aliphatiques, cycloaliphatiques, aromatiques, halogénoaromatiques, les esters benzyliques et les éthers aromatiques.

13. Procédé selon la revendication 12 caractérisé en ce que le solvant est choisi parmi le chlorobenzène et le diphényléther.

**Patentansprüche**

1. Verfahren zur Herstellung von aromatischen Säuren, das das Rückführen des Katalysators erlaubt, bei dem ein aromatisches Halogenid, dessen Halogen(e) Iod und/oder Brom sind, in Lösung in einem organischen Lösungsmittel, mit Kohlenoxid und Wasser in Gegenwart eines Katalysators auf der Basis von Palladium, einer organischen tertiären Stickstoffbase und eines Mittels, das mit Palladium einen Komplex bildet, ausgewählt aus den Phosphinen oder den Phosphiten, in Berührung gebracht wird, dadurch gekennzeichnet, daß das Reaktionsmedium aus einer organischen Phase und einer wäßrigen Phase besteht und daß die wäßrige Phase in ausreichender Menge vorhanden ist, um die Salze der organischen tertiären Stickstoffbase und der bei der Reaktion freigesetzten Säuren lösen zu können.

2. Verfahren nach Anspruch 1, dadurch gekennzeichnet, daß das aromatische Halogenid der Formel (I)

$$(X)_n - Ar - (R_1)_{n1} \ (I)$$

entspricht, in der
— X Chlor oder Brom bedeutet,
— Ar für eine mono- oder polycyclische oder heterocyclische Gruppe steht,
— n pro Ring 1 oder 2 ist,
— n1 eine ganze Zahl $\geq$ 1 und $\leq$ 4 ist,
— $R_1$ eine oder mehrere identische oder unterschiedliche Gruppen bedeutet, ausgewählt unter folgenden Gruppen: Wasserstoff, Fluor, Chlor, Nitril, Alkyl, Alkenyl, Alkoxy, Cycloalkyl, Cycloalkoxy, Alkylcarbonyloxy, Cycloalkylcarbonyloxy, Aryl, Aryloxy, Arylcarbonyloxy, Aryloxycarbonyl, wobei die Alkyl- und Arylgruppen 1 bis 20 Kohlenstoffatome aufweisen und gegebenenfalls mit einem oder mehreren Fluor- und/oder Chloratom(en) substituiert sind.

3. Verfahren nach Anspruch 2, dadurch gekennzeichnet, daß in der Formel (I) Ar für eine Benzol- oder Diphenylethergruppe steht.

4. Verfahren nach einem der Ansprüche 1 bis 3, dadurch gekennzeichnet, daß man unter den Verbindungen der Formel (I) p-Bromdiphenylether und 4'4-dibromdiphenylether verwendet.

5. Verfahren nach Anspruch 1, dadurch gekennzeichnet, daß der Katalysator aus metallischem Palladium oder einer Palladiumverbindung besteht.

6. Verfahren nach einem der Ansprüche 1 bis 5, dadurch gekennzeichnet, daß die Menge Palladium angegeben in g-Atom Edelmetall oder in mol Metallverbindung pro mol aromatisches Halogenid in einem Bereich von $10^{-5}$ bis $10^{-1}$ liegt.

7. Verfahren nach einem der Ansprüche 1 bis 6, dadurch gekennzeichnet, daß die Menge an Phosphorverbindung so beschaffen ist, daß das Verhältnis der Anzahl g-Atome Phosphor zur Anzahl g-Atome Metall in einem Bereich von 1 bis 10 000 liegt.

8. Verfahren nach Anspruch 1, dadurch gekennzeichnet, daß die tertiäre Stickstoffbase Triethylamin ist.

9. Verfahren nach Anspruch 1, dadurch gekennzeichnet, daß die Reatkionstemperatur 100 bis 250°C beträgt.

10. Verfahren nach Anspruch 1, dadurch gekennzeichnet, daß der Partialdruck des Kohlenmonoxids unter 5 bar liegt.

11. Verfahren nach Anspruch 10, dadurch gekennzeichnet, daß der Partialdruck des Kohlenmonoxids etwa 3 bar beträgt.

12. Verfahren nach Anspruch 1, dadurch gekennzeichnet, daß das Lösungsmittel aus den aliphatischen, cycloaliphatischen, aromatischen und halogenaromatischen Kohlenwasserstoffen, den Benzylestern und den aromatischen Ethern ausgewählt wird.

13. Verfahren nach Anspruch 12, dadurch gekennzeichnet, daß das Lösungsmittel aus Chlorbenzol und Diphenylether ausgewählt wird.

**Claims**

1. Process for the preparation of aromatic acids, enabling the catalyst to be recycled, a process which brings an aromatic halide in which the halogen or halogens are iodine and/or bromine in solution in an organic solvent into contact with carbon monoxide and water in the presence of a palladium-based catalyst, a tertiary nitrogenous organic base and a palladium-complexing agent chosen from phosphines or phosphites, characterized in that the reaction medium consists of an organic phase and an aqueous phase, the aqueous phase being in an amount sufficient to dissolve the salts of the tertiary nitrogenous organic base with the acids given off by the reaction.

2. Process according to claim 1, characterized in that the aromatic halide corresponds to the formula (I):

$$(X)_n - Ar - (R_1)_{n1} \ (I)$$

in which:
X denotes chlorine or bromine,
Ar denotes a mono- or polycyclic or heterocyclic radical,
n is equal to 1 or 2 per ring,
n1 is an integer greater than or equal to 1 and smaller than or equal to 4, and
$R_1$ denotes one or more identical or different groups chosen from the following radicals: hydro, fluoro, chloro, nitrile, alkyl, alkenylene, alkoxy, cycloalkyl, cycloalkoxy, alkylcarbonyloxy, cycloalkylcarbonyloxy, aryl, aryloxy, arylcarbonyloxy and aryloxycarbonyl, the alkyl and aryl moieties containing from 1 to 20 carbon atoms and, if desired, being substituted by one or more fluorine and/or chlorine atoms.

3. Process according to claim 2, characterized in that in the formula (I) Ar denotes a benzene moiety or diphenyl ether.

4. Process according to any one of claims 1 to 3, characterized in that, among the compounds of formula (I), parabromodiphenyl ether and di(4'4-bromophenyl) ether are employed.

5. Process according to claim 1, characterized in that the catalyst consists of metallic palladium or a palladium derivative.

6. Process according to any one of claims 1 to 5, characterized in that the quantity of palladium, expressed in gram-atoms of noble metal or in moles of metal derivative per mole of aromatic halide lies in a range from $10^{-5}$ to $10^{-1}$.

7. Process according to any one of claims 1 to 6, characterized in that the quantity of phosphorus derivative is such that the ratio of the number of gram-atoms of phosphorus to the number of gram-atoms of metal lies in a range from 1 to 10,000.

8. Process according to claim 1, characterized in that the tertiary nitrogenous base is triethylamine.

9. Process according to claim 1, characterized in that the reaction temperature is between 100 and 250°C.

10. Process according to claim 1, characterized in that the partial pressure of carbon monoxide is less than 5 bar.

11. Process according to claim 10, characterized in that the partial pressure of carbon monoxide is approximately 3 bar.

12. Process according to claim 1, characterized in that the solvent is chosen from aliphatic, alicyclic, aromatic and haloaromatic hydrocarbons, benzyl esters and aromatic ethers.

13. Process according to claim 12, characterised in that the solvent is chosen from chlorobenzene and diphenyl ether.